# EUROPEAN PATENT APPLICATION

(11) **EP 4 635 434 A1**
(43) Date of publication of application: **22.10.2025**
(21) Application number: 25164772.3
(22) Date of filing: 19.03.2025
(51) Int. Cl.: A61B 17/72

(54) **MOTORIZED INTRAMEDULLARY NAIL FOR BONE LENGTHENING OR TRANSPORT WITH IMPROVED POWER SUPPLY**

(30) Priority: 18.04.2024 IT 202400008893
(71) Applicant: Orthofix S.r.l., 37012 Bussolengo (VR) (IT)
(72) Inventor: VICENZI, Federico, 37136 Verona (IT); TOSONI, Simone, 25015 Desenzano del Garda (BS) (IT); LORENZINI, Denis, 37013 Caprino Veronese (VR) (IT); VENTURINI, Daniele, 37064 Povegliano Veronese (VR) (IT)
(74) Representative: Botti & Ferrari S.p.A.

(57) **Abstract**

A motorized intramedullary nail (1) for bone lengthening or transport, of the type comprising:
- a first tubular portion (2) extending in a prevalent axial direction of the intramedullary nail (1);
- a second rod-shaped portion (3) telescopically sliding inside the first tubular portion (2) and along the same axial direction;
- motorized electro-mechanical means (7) for driving the rod-shaped portion (3) which are inserted inside the first tubular portion (2) and comprising electrical terminals (12, 13) accessible through the proximal part of the nail (1);
characterized in that it further comprises:
- an independent power supply means (25) with biocompatible features for subcutaneous and prolonged implantation, connected and adapted to providing, preferably with an electronic control, electrical power according to the surgeon's plan, to operate said motorized electro-mechanical means (7), the independent power supply means (25) comprising a biocompatible casing (26) and at least one power supply battery (27) housed in the casing (26).

## Description

### Field of application

The present disclosure relates to the general technical field of orthopedics. More in particular, it relates to a motorized intramedullary nail for bone lengthening or transport of long bones.

For example, an intramedullary nail of this type allows to carry out a limb lengthening without using external fastening means; moreover, it may also be used for optimal transport and alignment of bone stumps or even for a desired block of a joint for the purposes of a post-traumatic or therapeutic treatment.

### Prior art

In this specific technical field, a type of motorized intramedullary nails allowing to carry out a lengthening of the long limbs is already known. For example, a system of this type called Fitbone^{™} has been known for many years, and was initially designed by Prof. Rainer Baumgart, a known world-famous specialist.

This system is mainly intended to lengthen even single limbs that require operations due to trauma or congenital malformations, or to lengthen limbs that are not completely developed in length.

Moreover, with a suitable preoperative planning, an intramedullary nail of the previously indicated type allows to provide axial and torsional corrections as a part of limb lengthening.

Said nails are configured with a first portion which can be defined as fixed and a second portion which is telescopically sliding and extensible inside the first fixed portion.

Motorized driving electro-mechanical means are provided to control the lengthening of the sliding portion by means of externally applied control signals.

For example, a prior-art document, no. WO 2021/032823 A1, relates to a medullary nail for lengthening a long bone comprising a first tubular body and a second tubular body which is inserted in the first tubular body and sliding inside it, and also connected by means of gears to a small electrical motor which allows an axial translation thereof relative to the first tubular body.

Although they are advantageous under several aspects, said known nails for bone lengthening have some drawbacks.

In particular, the small electrical motor nested inside the nail must be powered by electric current.

In the oldest solutions, it was necessary to provide external cables, insertable up to the prosthesis to provide the related voltage and to allow the nail to function.

In the most modern solutions, it is possible to use a technology of the inductive type to avoid this very invasive type of operation.

However, this type of solution also forces the user to periodically position himself/herself at the power supply so as to obtain the desired lengthening, which clearly limits quite a bit the patient's freedom of movement.

The technical problem underlying the present disclosure is to provide a new type of power supply for motorized nails or plates, such as, for example, a motorized trochanteric intramedullary nail having such structural and functional features to allow overcoming all the previously mentioned drawbacks.

A further object is to provide a solution that can be easily inserted and can be easily operated by both the surgeon and the patient.

Another further object is to provide a safe and durable solution so as not to negatively affect the patient's life.

Clearly, an object of the invention is also to provide a solution in accordance with the provided surgical and medical safety requirements.

Finally, an object of the present solution is also to be applicable, possibly with slight modifications, also to preexisting devices.

### Summary of the invention

The solution idea underlying the invention is to provide a different type of power supply for an intramedullary nail, so that the patient does not require anymore to be near or at an external power supply.

According to this solution idea, the present disclosure relates to a motorized and improved intramedullary nail improved for bone lengthening or transport, of the type comprising a first tubular portion extending in a prevalent axial direction of the intramedullary nail, a second rod-shaped portion telescopically sliding inside the first tubular portion and along the axial direction, and motorized electro-mechanical means for driving the rod-shaped portion which are inserted inside the first tubular portion and comprising electrical terminals accessible through the proximal part of the nail.

The nail according to the present invention further comprises an independent power supply means with biocompatible features for subcutaneous and prolonged implantation, connected and adapted to providing electrical power to the motorized electro-mechanical means.

Advantageously, the present solution allows, during implantation of the nail, to also apply the related means of power supply and management of the internal nail which allows to electrically power and thus to activate nail operation in any place, independently of the presence of designated external power supplies.

Specifically, the independent power supply means comprises a biocompatible casing and at least one power supply battery housed in the casing.

The above-mentioned casing may be, for example, a casing similar to a pacemaker casing.

Advantageously, said solution allows to independently develop the best biocompatible material for the casing and the best battery to optimize performance.

More preferably, the power supply battery is a lithium battery.

Advantageously, lithium is the element with the highest specific energy, and it is thus to this day the most adopted element to build batteries, thereby ensuring greater longevity.

Inside the batteries, conductivity is promoted by adding solvents, such as acetonitrile, and solutes, in addition to other materials, such as sulfides, for the active cathode.

Lithium batteries are widely used in pacemakers, since they meet the requirements of longevity, low discharge voltage and low discharge current.

The requirement of longevity is met thanks to the fact that the lithium metal surface is passivated through the reaction with the electrolyte.

All lithium systems are said to be thermodynamically unstable and kinetically stable, do not produce gas and can thus be hermetically sealed.

The pressure drop, which is characteristic of the discharge terminal, behaves in the best way, decreasing quite slowly, and allowing to recognize the end of the battery's life cycle.

More preferably, the power supply battery is a lithium hydride battery or a lithium iodine battery.

Advantageously, lithium hydride batteries have an initial open-circuit voltage of 2.8V, which is thus still lower than the maximum of 3V that is generally allowed for a subcutaneous implant, and an even ten-year duration.

Lithium iodine batteries are solid-electrolyte batteries, with the anode consisting of lithium (or of lithium incorporated in carbon material such as graphite) and the cathode consisting of iodine and of the polymer poly-2-vinylpyridine.

Advantageously, these provide a bigger volume and a greater resistance and do not have potential leakage, have high reliability and a longer lifetime. A little drawback is the limitation of the battery's output current.

Nothing prevents to provide different solutions such as lithium cupric sulfide batteries, or batteries in advanced development stage such as lithium carbon monofluoride batteries, (liquid-electrolyte) lithium polycarbonate fluoride batteries, or also and thin film batteries (TFB) which take advantage of the properties of lithium polymer.

Preferably, the casing of the power supply means is a metal casing, for example made of titanium, or a silicone casing.

Advantageously, a metal casing, for example made of titanium, has a good fracture resistance, excellent strength, and compatibility with the human body with bone-bonding and bone-formation capabilities and an excellent corrosion strength. Silicone is also a material which is compatible with the human body, easier to handle and to shape, and lighter.

More preferably, the casing provides a maximum transversal dimension between about 20 mm and 50 mm, a maximum longitudinal dimension between about 20 mm and 50 mm, and a maximum depth between about 4 mm and 10 mm.

Advantageously, the present solutions are optimal for the application without the need for further severing compared to the widely adopted surgical technique.

In a preferred embodiment, the motorized electro-mechanical means are coupled to a control unit which is electrically powered and inserted in said casing, which is as described for example similar to the one used in pacemakers, said control unit being adapted to provide power according to a selected operating mode to the electro-mechanical means.

Advantageously, the present solution allows the surgeon to set the power delivery based on patient-specific schedules and ranges according to the rehabilitation therapy considered the most suitable.

Moreover, advantageously, remote actuation by the surgeon could be also possible in a post-operational therapy phase according to the ways planned with the patient.

More preferably, the intramedullary nail also comprises a plurality of displacement sensors, and the control unit is programmable and functionally connected to said plurality of sensors.

The control unit is thus adapted for the collection of raw data from the plurality of sensors, the internal processing of said data and sending via a communication protocol to the outside, generally to a device of the surgeon, for the detection of a lengthening or shortening of the intramedullary nail.

Also preferably, the motorized electro-mechanical means comprise an electrical motor or gearmotor and a screw drive.

Advantageously, the present solution is efficient and allows a fine adjustment of the lengthening of the intramedullary nail.

More preferably, the screw drive comprises a endless screw and a nut screw, the nut screw being operable in translation with respect to the endless screw, the second rod-shaped portion being integral in translation with the nut screw and the first tubular portion being integral in translation with the endless screw, or vice versa.

Advantageously, the present solution is structurally robust and precise as regards adjustment.

Preferably, the intramedullary nail is of trochanteric type with a proximal part with centrally obtained transverse and tilted holes for trochanteric screws.

Advantageously, the present solution is particularly suitable for applications in long bones.

Also preferably, the motorized electro-mechanical means for driving the rod-shaped portion are housed in a cartridge or capsule having a bottom or distal wall from which the electrical terminals emerge.

Advantageously, the present solution is suitable for the implementation in preexisting solutions.

The features and advantages of the motorized intramedullary nail according to the present disclosure will become apparent from the following description of an embodiment, given by way of non-limiting example with reference to the accompanying drawings.

### Brief description of the drawings

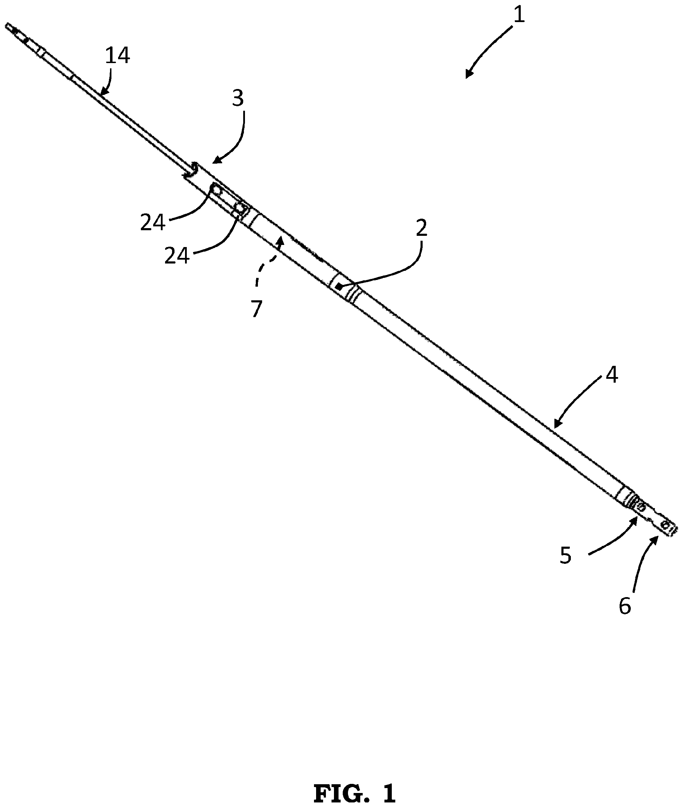

- Figure 1 shows a perspective view of a non-powered intramedullary nail adopted in the present invention;
- Figure 2 shows a sectional view of the intramedullary nail of Figure 1;

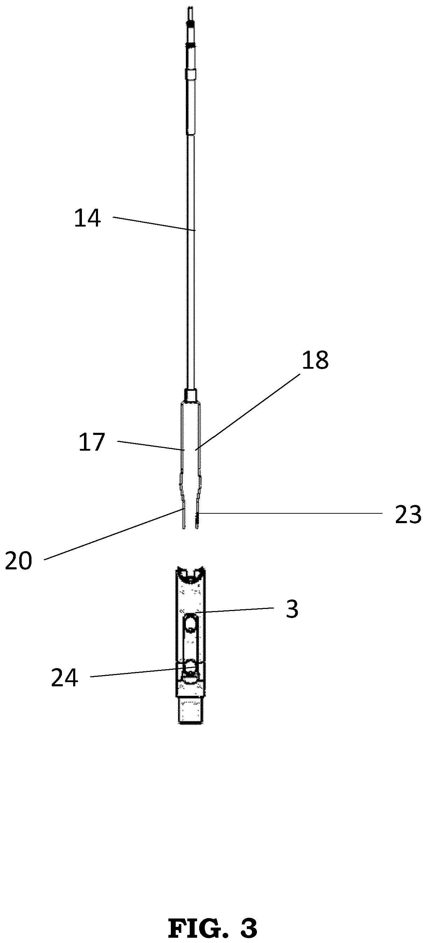

- Figure 3 shows a front view of the intramedullary nail of Figure 1;
- Figure 4 shows a view of a detail of the intramedullary nail of Figure 1;
- Figure 5 shows a perspective view of a first application of intramedullary nail powered according to the present invention, and Figure 5A is an enlargement of a related detail;
- Figure 6 shows a perspective view of a second application of intramedullary nail powered according to the present invention;
- Figure 7 shows a perspective view of a third application of intramedullary nail powered according to the present invention.

### Detailed description

With reference to those figures, reference number 1 globally and schematically illustrates a preferred embodiment of the intramedullary nail 1 improved according to the present disclosure to carry out bone lengthening of a long bone as needed. For these purposes, the nail 1 is substantially an extensible nail and, as will be described below, is extensible through the activation of electro-mechanical driving means inserted in said nail.

The intramedullary nail 1, in the non-limiting exemplary embodiment shown, is of the trochanteric type, meaning that it is particularly suitable for femoral use and may have a proximal part slightly tilted with respect to the prevalent longitudinal axis of said nail. However, it is also possible that the nail is also usable for a tibia or a humerus, in any case, for a bone that can be defined as long.

As it will be described hereinafter, the intramedullary nail 1 may be coupled to an electro-mechanical actuator or gearmotor equipped with a signal receiver or of an incorporated control unit. External control electronics and a first communication device may also be provided. Thus, the commands necessary for the nail-lengthening process are sent to the second communication device, without any contact between the implanted intramedullary nail 1 and the surface (skin) of the body.

The intramedullary nail 1 actually comprises a first tubular portion 2 which can be defined as fixed and which extends in an axial direction of said nail. Preferably, this portion may have a circular section and comprises a proximal part 4 which is generally slightly tilted with respect to the prevalent longitudinal axis of the nail 1.

The terms "axial" and "radial" must be intended herein with respect to the longitudinal axis of the intramedullary nail 1; that is, an axis that extends according to the prevalent length of the intramedullary nail 1 or anyway in the direction of maximum spatial expansion of the intramedullary nail 1.

More in particular, axial direction indicates a direction that is aligned or parallel to the longitudinal axis of the intramedullary nail 1, whereas radial direction indicates a direction that is perpendicular to the longitudinal axis.

It should be noted that the longitudinal axis of the trochanteric intramedullary nail 1 has generally a slightly tilted portion; for example, a slight curvature of the proximal part 4 that promotes the correct positioning of transverse trochanteric screws that must penetrate a femoral bone with a trochanteric neck tilted by about 30° with respect to the mediolateral plane. This slightly tilted portion is missing, for example, in other nails for other tibial and humeral applications.

Typically, the tubular portion 2 is made of metal or of a metal alloy, in particular a biocompatible metal or a biocompatible metal alloy. Instead, other elements of the intramedullary nail 1 are made of a synthetic-plastic material, for example epoxy resin, silicone or thermoplastic resin.

The tubular portion 2 comprises a hollow cylindrical shell inside which a second rod-shaped portion 3, which may be in turn tubular, is slidingly and telescopically mounted. This rod-shaped portion 3 may also be made with multiple interconnected stages.

The rod-shaped portion 3 comprises, in distal position, a first hole 5 that is transverse to the axial direction and configured to house a bone screw. Alternatively, the hole 5 may be threaded to house a guide screw to prevent the rotation of the rod-shaped portion 3 with respect to the tubular portion 2.

A second hole 6 that is transverse to the axial direction and that can be parallel to the first hole 5 or tilted, depending on the fixing needs of the rod-shaped portion 3, is also provided.

Motorized electro-mechanical means 7 are further provided to operate in translation the rod-shaped portion 3 inside the tubular portion 2.

Essentially, the intramedullary nail 1 comprises motorized means 7 configured to move the rod-shaped portion 3 inside the tubular portion 2 axially and with millimeter precision. Preferably, the above-mentioned means 7 comprise, for example, an electrical motor or gearmotor 8 and a screw drive 9.

The electrical motor 8 is adapted to operate the screw drive 9, which in turn is coupled and acts on the rod-shaped portion 3 for a reversible displacement thereof.

Specifically, the electrical motor 8 provides the rotary movement to the screw drive 9 coupled to the rod-shaped portion 3 for the reversible displacement of said rod-shaped portion 3.

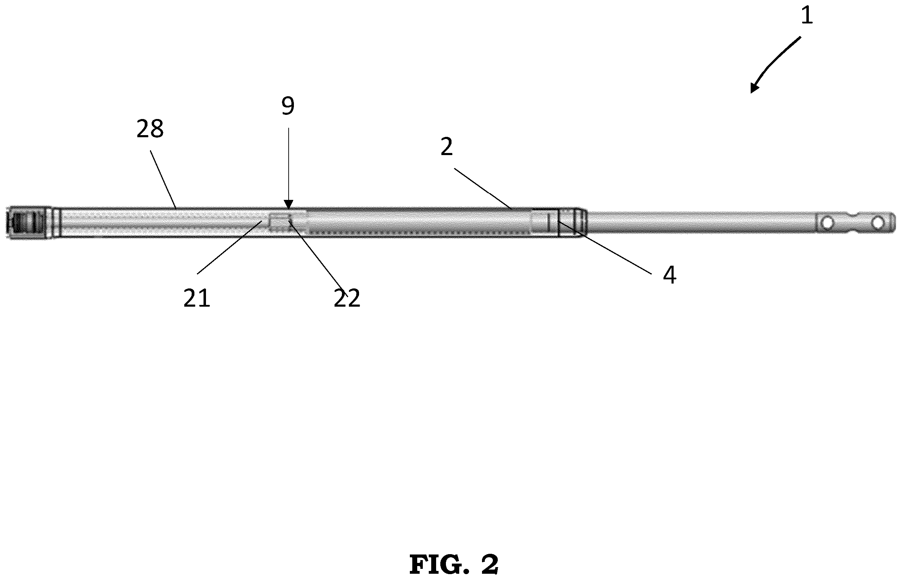

More specifically, the screw drive 9, as shown in Figure 2, is consists of a endless screw 21 and a nut screw 22 coupled thereto. The endless screw 21 is rotatably mounted inside the tubular portion 2, integral in translation with the same and coaxial thereto. Instead, the nut screw 22 is integral with the end of the rod-shaped portion 3, and is sliding within a guide segment 28 of the tubular portion 2 that prevents the relative rotation thereof. The motor 8, coupled to the endless screw 21, is configured to put it in rotation, thereby promoting the displacement in the longitudinal direction x of the nut screw 22 and of the rod-shaped portion 3 integral therewith.

The electrical motor 8 is housed inside a protection cartridge or capsule 10 which also houses electronic control means (not shown) of the motor 8 and which is inserted into the proximal part 4 of the nail 1 after insertion of the rod-shaped portion 3.

Thus, in the present embodiment, the motorized electro-mechanical means 7 are also remotely actuatable, which enables them to also be remotely actuated by the surgeon in the post-operational therapy phase according to the ways planned with the patient.

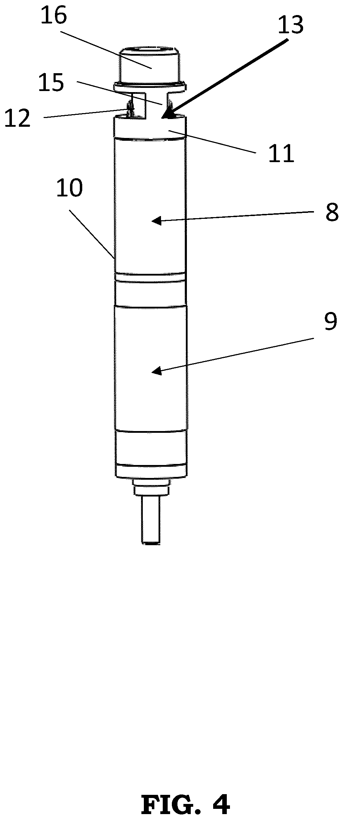

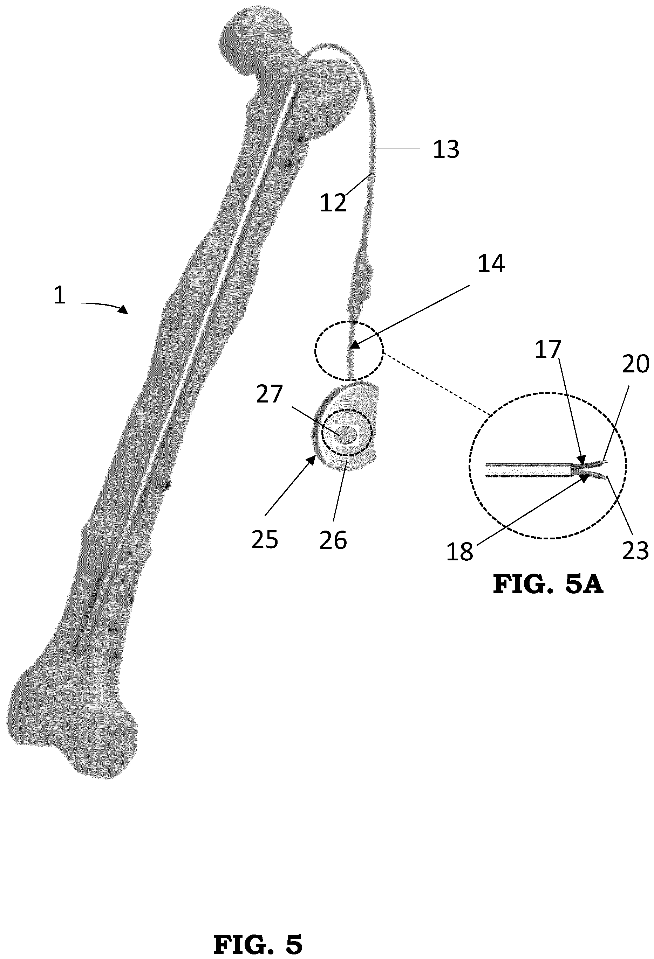

As shown in Figure 4, in the present non-limiting exemplary embodiment, the electrical-power terminals 12, 13 emerge from the capsule 10 through a base or bottom wall 7 on the side facing the bipolar electrical-power cable 14. The bipolar cable 14 comprises two electrical conductors 17, 18, shown in Figure 5A, having respective ends 20, 23 for connecting to the terminals 12, 13 of connection to the motor 8.

A separation and support partition 15 for a covering element 16 which also carries out the function of protecting said terminals 12, 13 is provided between these terminals 12 and 13. The partition 15 is also a spacer for supporting the covering element 16 which keeps it in a preset spaced-apart relationship with the base wall 11 of the capsule 10.

Considering the proximal portion of the capsule 10 as if it were stand-alone, we could say that the base wall 11 from which the terminals 12 and 13 emerge is covered by a roof represented by the covering element 16 and centrally supported by the partition 15 which also separates the two terminals 12, 13 from each other, as if it were a separation partition. The partition 15 has lateral dimensions such as to occupy the same lateral space as the capsule 10, whereas the covering element 16, substantially circular, has the same overall dimensions as the bottom wall 11 of the capsule 10.

The separation and support partition 15 and the covering element 16 are made of an electrically insulating material.

Even the capsule 10 is made of an insulating material and the rear or proximal wall 7 of the capsule 10 may be in a single piece made of the same insulating material as the partition 15 and the covering element 16.

As shown in Figures 5-7, the motor 8, according to the present invention, is powered through an independent power supply means 25 with biocompatible features for subcutaneous and prolonged implantation, connected and capable of operating the motorized electro-mechanical means 7.

In particular, Figure 5 shows an embodiment of the intramedullary nail 1 with the proximal end 3, and the related motorized electro-mechanical means 7 facing the head of a femur, from which the terminals 12, 13 come out, said terminals being connected to the electrical conductors 17, 18 which in turn come out from the independent power supply means 25.

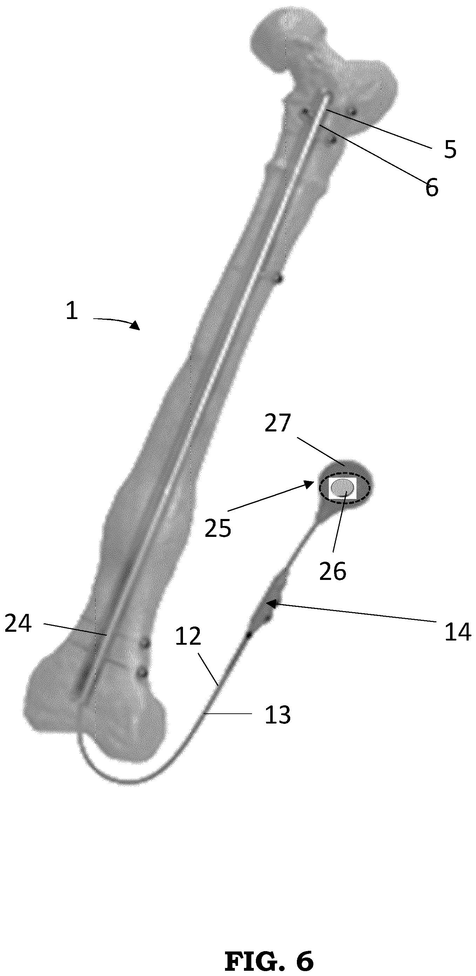

In Figure 6, the intramedullary nail 1 is inserted in an inverted position, thus with the proximal end 3, and the related motorized electro-mechanical means 7 facing the distal epiphysis of a femur, from which similarly the terminals 12, 13 come out, said terminals being connected to the electrical conductors 17, 18 which in turn come out from the independent power supply means 25.

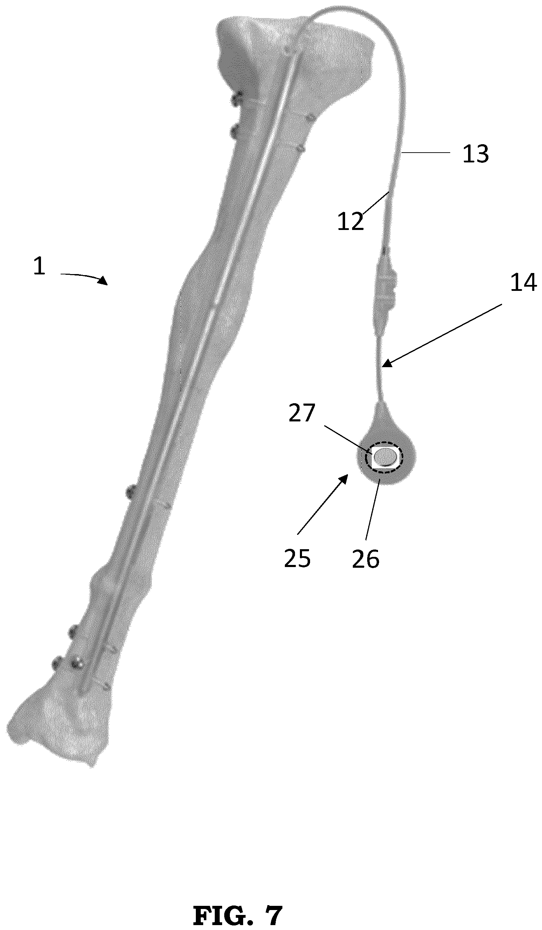

Instead, in Figure 7, the intramedullary nail 1 is inserted inside a tibia.

Nothing prevents to adopt the present solution also in other applications, with relative specific modifications, which are obvious depending on the anatomical variation.

During implantation of the intramedullary nail 1, the related independent power supply means 25 is also applied inside the involved limb, which fact allows to activate the operation of the intramedullary nail 1 in any place, regardless of the presence of designated external power supplies.

According to the present invention, the independent power supply means 25 comprises a biocompatible casing 26 and at least one power supply battery 27 housed in the casing 26.

The casing 26 provides a maximum transversal dimension between about 20 and 50 mm, a maximum longitudinal dimension between about 20 and 50 mm, and a maximum depth between 4 and 10mm.

These size ranges are optimal for the application on long bones as is shown in Figures 5-7, without the need for further severing compared to the widely adopted surgical technique.

The subdivision of the power supply means 25 in these two components, with the power supply battery 27 in the welded casing 26, allows to use the best material in terms of biocompatibility for the casing 26 and the best power supply battery 27 in terms of performance.

In the present embodiment, the power supply battery 27 is a lithium battery, since lithium is the element with the highest specific energy and greatest longevity.

Inside lithium batteries, conductivity is promoted by adding solvents, such as acetonitrile, and solutes, in addition to other materials, such as sulfides, for the active cathode.

Lithium batteries are widely used in pacemakers, precisely because they meet the requirements of longevity, low discharge voltage and low discharge current.

The requirement of longevity is met thanks to the fact that the lithium metal surface is passivated through the reaction with the electrolyte.

All lithium systems are said to be thermodynamically unstable and kinetically stable, do not produce gas and can thus be hermetically sealed, as in the case of pacemakers and as in the present case with the power supply battery 27 introduced into the casing 26.

The pressure drop, which is characteristic of the discharge terminal, behaves in the best way, decreasing quite slowly, and allowing to recognize the end of the battery's life cycle.

Preferably, depending on the type of application, on the necessary costs, and on possible particular needs of the patient or of the surgeon, the power supply battery 27 may be a lithium hydride battery or a lithium iodine battery.

Advantageously, lithium hydride batteries have an initial open-circuit voltage of 2.8V, which is thus still lower than the maximum of generally 3V that is allowed for a subcutaneous implant, and an even ten-year duration.

Lithium iodine batteries are solid-electrolyte batteries, with the anode consisting of lithium (or of lithium incorporated in carbon material such as graphite) and the cathode consisting of iodine and of the polymer poly-2-vinylpyridine.

Advantageously, these a bigger volume and a greater resistance and do not have potential decay, have high reliability and a longer lifetime. At most, a little drawback is the limitation of the battery's output current.

It is also possible to provide different solutions such as lithium cupric sulfide batteries, or batteries in advanced development stage such as lithium carbon monofluoride batteries, (liquid-electrolyte) lithium polycarbonate fluoride batteries, or also and thin film batteries (TFB) which take advantage of the properties of lithium polymer.

Preferably, the casing 26 of the power supply means 25 is a metal casing, for example made of titanium, or a silicone casing.

Advantageously, a metal casing, for example made of titanium, as it is known in the medical field, has a good fracture resistance, excellent strength, and compatibility with the human body with bone-bonding and bone-formation capabilities and an excellent corrosion strength. Silicone is also a material which is compatible with the human body, easier to handle and to shape, and lighter.

Moreover, in the present embodiment, the casing 26 also comprises inside it the above-mentioned control unit (not shown), which is adapted to provide power according to a selected operating mode to the electro-mechanical means 7. The control unit may be powered through said power supply battery 27.

This allows the surgeon to set the power delivery based on patient-specific schedules and ranges according to the rehabilitation therapy considered the most suitable.

More preferably, the intramedullary nail 1 also comprises a plurality of displacement sensors (not shown), and the control unit is programmable and functionally connected to said plurality of sensors.

Thus, the control unit is adapted for the collection of raw data from the plurality of sensors, the internal processing of said data and sending via a communication protocol to the outside, generally to a device of the surgeon, for the detection of a lengthening or shortening of the intramedullary nail 1.

The communication protocol may be one of those available in the field.

In other words, the present preferred embodiment also provides to integrate the above-mentioned plurality of sensors to be able to verify what is happening on the intramedullary nail 1, and consequently to the limb-lengthening process, to enable the surgeon to possibly intervene on the treatment.

Thus, in this case, the surgeon has access to the external control electronics and to the first communication device, and the internal control unit comprises the second communication device for exchange of information.

Nothing prevents to provide, in very specific cases, an intramedullary nail 1 with a control unit programmed before implantation and having all the commands of response to the variations detected in the intramedullary nail 1, without the need for any communication with the outside.

In general, once implanted, the intramedullary nail 1 with the power supply means 25 placed at a cavity which is in any case present as a consequence of the surgical technique already widely adopted in the field, the patient is advantageously freed from the presence of an external power supply to carry out the planned lengthening of said intramedullary nail at a millimeter level.

Advantageously, as it can be appreciated, the present solution is not particularly complex to be implemented, so it does not entail particular drawbacks during the production phase.

Moreover, advantageously, the present solution takes advantage of well-known properties of some components, to create an efficient, safe, and robust solution to solve a current significant limitation.

The person skilled in the art will understand that the described embodiment may be subjected to various modifications and variations, depending on particular needs and specifications, all included in the protection scope of the invention, as defined by the following claims.

For example, nothing prevents to provide a different conformation of intramedullary nail, a different type of power supply battery or the use, for example of specific anchoring devices for the independent power supply means.

Moreover, nothing prevents to apply the present invention to motorized plates.

Furthermore, nothing prevents to provide the use of different materials for the intramedullary nail or for the casing of the power supply means or different sizes for different applications.

## Claims

1. A motorized intramedullary nail (1) for bone lengthening or transport, of the type comprising:
- a first tubular portion (2) extending in a prevalent axial direction of the intramedullary nail (1);
- a second rod-shaped portion (3) telescopically sliding inside said first tubular portion (2) and along said axial direction;
- motorized electro-mechanical means (7) for driving the rod-shaped portion (3) which are inserted inside said first tubular portion (2) and comprising electrical terminals (12, 13) accessible through the proximal part (4) of the nail (1);
**characterized in that** it further comprises:
- an independent power supply means (25) with biocompatible features for subcutaneous and prolonged implantation, connected and adapted to providing electrical power to operate said motorized electro-mechanical means (7),
wherein said independent power supply means (25) comprises a biocompatible casing (26) and at least one power supply battery (27) housed in said casing (26).

2. Motorized intramedullary nail (1) according to claim 1, wherein said power supply battery (27) is a lithium battery.

3. Motorized intramedullary nail (1) according to claim 2, wherein said power supply battery (27) is a lithium hydride battery or a lithium iodine battery.

4. Motorized intramedullary nail (1) according to any one of claims 1 to 3, wherein said casing (26) is a metal or silicone casing.

5. Motorized intramedullary nail (1) according to claim 4, wherein said casing (26) provides a maximum transversal dimension between about 20 mm and 50 mm, a maximum longitudinal dimension between about 20 mm and 50 mm, and a maximum depth between about 4 mm and 10 mm.

6. Motorized intramedullary nail (1) according to any one of claims 1 to 5, wherein said motorized electro-mechanical means (7) are coupled to a control unit which is electrically powered and inserted in said casing (26), adapted to provide power according to a selected operating mode to said electro-mechanical means (7).

7. Motorized intramedullary nail (1) according to claim 6, further comprising a plurality of displacement sensors, said control unit being programmable and functionally connected to said plurality of sensors for the collection of raw data from said plurality of sensors, the internal processing of said data and sending via a communication protocol to the outside, for the detection of a lengthening or shortening of said intramedullary nail (1).

8. Motorized intramedullary nail (1) according to any one of claims 1 to 7, wherein said motorized electro-mechanical means (7) comprise an electrical motor or gearmotor (8) and a screw drive (9).

9. Motorized intramedullary nail (1) according to claim 8, wherein said screw drive (9) comprises a endless screw (21) and a nut screw (22), said nut screw (22) being operable in translation with respect to said endless screw (21), said second rod-shaped portion (3) being integral in translation with said nut screw (22) and said first tubular portion (2) being integral in translation with said endless screw (21), or vice versa.

10. Motorized intramedullary nail (1) according to any one of claims 1 to 9, wherein the intramedullary nail (1) is of the trochanteric type with a proximal part (4) with centrally-obtained transversal and tilted holes (24) for trochanteric screws.

11. Motorized intramedullary nail (1) according to any one of claims 1 to 10, wherein said motorized electro-mechanical means (7) for driving the rod-shaped portion (3) are housed in a cartridge (10) or capsule having a bottom or distal wall (11) from which the electrical terminals (12, 13) emerge.
